# EUROPÄISCHE PATENTSCHRIFT

(11) **EP 2 228 024 B1**
(45) Veröffentlichungstag und Bekanntmachung des Hinweises auf die Patenterteilung: **07.05.2014**
(21) Anmeldenummer: 10156161.1
(22) Anmeldetag: 11.03.2010
(51) Int. Cl.: A61B 17/34, A61B 17/02

(54) **Medizinisches Instrument zum Schaffen eines Zugangs für einen minimalinvasiven Eingriff**
Medical instrument for creating an access point for minimally invasive procedures
Instrument médical destiné à créer un accès pour une intervention mini-invasive

(30) Priorität: 13.03.2009 DE 102009014525; 17.04.2009 DE 102009018639
(43) Veröffentlichungstag der Anmeldung: 15.09.2010
(73) Patentinhaber: Karl Storz GmbH & Co. KG, 78532 Tuttlingen (DE)
(72) Erfinder: Wagner, Sebastian, 75015 Bretten (DE); Teufel, Felix, 78532 Tuttlingen (DE)
(74) Vertreter: Witte, Weller & Partner Patentanwälte mbB

(56) Entgegenhaltungen:
- DE-A1- 2 801 696
- US-A- 4 432 351
- US-A- 5 580 344
- US-A1- 2004 002 629
- US-A1- 2004 093 001
- US-A1- 2006 235 279
- US-A1- 2007 027 364
- US-A1- 2007 219 416
- US-A1- 2008 234 550
- US-A1- 2008 255 519

## Beschreibung

Die Erfindung betrifft ein medizinisches Instrument zum Schaffen eines Zuganges für einen minimalinvasiven Eingriff, mit einem Körper, der über eine Mittellängsachse gesehen, aus zumindest zwei Teilkörpem zusammengesetzt ist, wobei jeder Teilkörper einen distalen Teilkörperabschnitt aufweist, der in einen von der Mittellängsachse nach außen abstehenden proximalen Teilkörperabschnitt übergeht, wobei die distalen Teilkörperabschnitte in einer ersten Position zu einem distalen Körper mit seitlich abstehenden proximalen Teilkörperabschnitten zusammenfügbar sind, und die proximalen Teilkörperabschnitte in einer zweiten Position zu einem proximalen Hohlkörper mit seitlich abstehenden distalen Teilkörperabschnitten zusammenfügbar sind, wobei ein Übergang von der ersten Position zur zweiten Position und umgekehrt durch Verschwenken der Teilkörper bewerkstelligbar ist, und dass die Teilkörperabschnitte, in Richtung der Mittellängsachse gesehen, Kantenflächen aufweisen, längs derer die Teilkörper zusammenfügbar sind.

Ein derartiges medizinisches Instrument ist aus der US 2006/235279 A1 bekannt.

Die beiden Teilkörper liegen über glatte Kantenflächen bündig aneinander an. In der ersten Position sind die beiden distalen Teilkörperabschnitte zu einem distalen Körper zusammengefügt, der über einen Einschnitt in der Haut in den menschlichen Körper eingetrieben werden kann, um Zugang zu einem Hohlraum im Inneren des menschlichen Körpers zu erhalten.

In der zweiten Position, sind die im Inneren des Körpers eingeschobenen distalen Teilkörperabschnitte gespreizt und die vom Körper abstehenden äußeren proximalen Teilabschnitte sind zu einem proximalen Hohlkörper zusammengefügt.

Durch diesen proximalen Hohlkörper können dann Instrumente in den Körper eingeführt werden.

Insbesondere bei der Laparoskopie wird der Bauchraum durch ein Gas aufgebläht, das durch den proximalen Hohlkörper hindurch eingeführt wird. Daher sollte der proximale Hohlkörper, über seinen Umfang gesehen, möglichst dicht sein.

Aus der US 4 432 351 A ist ein Scheidenspekulum bekannt, das aus zwei Teilkörperabschnitten zusammengesetzt ist, die durch ein diese umgebendes Ringelement vor seitlichem Abgleiten gehalten sind. Die Längskantenflächen der Teilkörper, an denen diese aneinanderliegen, sind mit einer reibenden Oberfläche versehen. Diese reibende Oberfläche der Kantenflächen kann durch eine Querverzahnung erfolgen, oder dass diese klebrig oder magnetisch gemacht werden. Dadurch soll erzielt werden, dass, falls mit der Hand nur auf einen Teilkörperabschnitt eine Kraft in Vorschubrichtung aufgeführt wird, der andere Teilkörperabschnitt aufgrund der reibenden Oberfläche der Kantenflächen axial mitgenommen wird.

Aus der US 2004/0002629 A1 ist ein Instrument für die minimalinvasive Chirurgie bekannt, bei der der hohlzylindrische Körper, der in den Körper des Patienten eingetrieben werden soll, aus zwei Halbschalen zusammengesetzt ist. Nachdem der hohlzylindrische Körper in den Patienten eingeführt ist, kann er durch einen Spreizmechanismus seitlich aufgespreizt werden, so dass die beiden Hohlkörper nicht mehr aneinanderliegen. Die Kantenflächen längs der die Halbschalen aneinanderliegen, sind glatt ausgebildet und können direkt aneinanderstoßen oder einander überlappen. Die Überlappung kann so ausgestaltet sein kann, dass sich die Wände über ihre komplette Wandstärke überlappen, oder, dass entsprechende Ausnehmungen im Fügebereich vorhanden sind, die zu dünneren Wandstärken führen, so dass dann die beiden Teilkörper über diese dünneren Wandkantenflächen überlappend aneinanderlegbar sind.

Aus der US 2004/093001 A1 ist ein medizinisches Zugangsinstrument bekannt, das aus halbschalenförmigen Teilkörpern zusammengesetzt ist. Die Teilkörper sind über deren Längskantenflächen zusammengesetzt. Von der radial äußeren Kantenlinie eines Teilkörpers springen Gebilde vor, die die Außenseite des anderen Teilkörpers übergreifen. Diese Gebilde sperren ein seitliches Abgleiten der Teilkörper quer zu den Kantenflächen.

Insbesondere in der Laparoskopie, in der minimalinvasive Eingriffe sehr verbreitet Einsatz gefunden haben, erfolgt dieser Zugang über sog. Trokare. Ein Trokar besteht aus einer Trokarhülse, die ein etwa 10 cm langes Rohr aufweist, an dessen proximalem Ende ein Gehäuse angeordnet ist. In dem Gehäuse sind meistens Ventile angeordnet, um gegenüber seitlichen Anschlüssen im Gehäuse abzudichten, über die bspw. Gase zum Aufblähen des Bauchraumes oder Flüssigkeiten zum Spülen geführt werden können. Am proximalen Ende ist das Trokargehäuse mit einer Dichtung versehen, die meist eine mittige Öffnung aufweist, durch die, nach Setzen der Trokarhülse, Instrumente in den Körper eingeschoben werden können, bspw. Endoskope zur visuellen Beobachtung oder sonstige Operationsinstrumente.

Zum Setzen des Trokars wird in die Trokarhülse von proximal ein Trokardorn eingeschoben, der so ausgebildet ist, dass dessen meist dreieckig ausgeformte scharfe Spitze gerade über das distale Ende der Trokarhülse hinausragt. Es wird eine kleine Inzision mit einer Länge von ca. 1 cm in der Haut durchgeführt, daran wird der Trokar bzw. die Spitze des von dem distalen Ende der Trokarhülse vorspringenden Trokardorns angesetzt und dann wird der Zusammenbau durch die Bauchdecke hindurchgedrückt. Danach wird der Trokardorn abgezogen und die Trokarhülse ist nunmehr frei, um durch diese hindurch einen minimalinvasiven Eingriff durchzuführen.

Bei der Laparoskopie muss die Trokarhülse durch die Bauchdecke hindurchgeschoben werden, die bei normal gebauten Menschen wenige Zentimeter dick ist. Am proximalen Ende der Trokarhülse werden oftmals mehrere Schläuche angeschlossen und manche Instrumente, bspw. chirurgische Zangen ragen sehr weit über das proximale Ende der Trokarhülse hinaus.

Dadurch wirken erhebliche Kippmomente auf die im Körper steckende Trokarhülse ein mit der Gefahr, dass der Trokar aus der ursprünglichen Ausrichtung seitlich verkippt.

Da die angeschlossenen Gerätschaften bei hochstehenden Trokaren auch eine Kraft in Gravitationsrichtung ausüben, kann es vorkommen, dass sich die Trokarhülse ungewünscht axial verschiebt. Ein Verschieben in distaler Richtung kann vom Operateur in einem gewissen Maße durch Ziehen am Trokar ausgeglichen werden. Wird die Trokarhülse aber zu weit nach proximal abgezogen, bspw. weil ein Instrument, das durch die Trokarhülse abgezogen werden soll, sich mit dem distalen Ende der Trokarhülse verhakt, so besteht die Gefahr, dass der gesamte Trokar aus dem Körper herausrutscht.

Noch stärker wirkt sich dieses Kippmoment aus, wenn Operationen durchgeführt werden, in denen der Trokar nicht vertikaler Richtung, sondern in schräger oder gar horizontaler Richtung eingebracht wird.

Es wurden daher Versuche unternommen, derartige Zugangsinstrumente fester oder besser am Körper zu halten.

Aus der US 3,717,151 ist eine Zugangskanüle in Form eines Trokares bekannt, die am distalen Ende ausklappbare Finger aufweist, die zum Einschieben in den Körper in einer axialen Richtung ausgerichtet sind und nach Durchstechen durch die Bauchdecke durch einen Spreizmechanismus seitlich ausgespreizt werden können. Vor dem Abziehen der Hülse müssen dann die gespreizten Finger wieder eingezogen werden, so dass die Kanüle vom Körper wieder abgezogen werden kann.

Diese Konstruktion verlangt eine aufwändige Mimik oder Mechanik zum Steuern der ausklappbaren Finger.

In der US 2008/0234550 A1 ist ein minimalinvasives Zugangsportal beschrieben, bei dem die Hülse, die in den Körper eingeschoben wird, seitlich aufschwenkbare Platten aufweist, die zum Einführen zu einem schlanken Körper verschwenkt werden können und dann im Körper über einen Spreizmechanismus aufgespreizt werden können.

Nachteilig an diesen Vorrichtungen ist, dass eine aufwändige Mimik bzw. Mechanik zum Spreizen und Einziehen der spreizbaren Elemente vorhanden sein muss, die einen komplizierten konstruktiven Aufbau erfordert.

Dieser Aufbau beinhaltet unzählige Ecken und Nischen, in denen sich Bakterien festsetzen können, so dass diese Vorrichtungen nur sehr aufwändig zu reinigen sind, bzw. vor jedem Reinigungsvorgang zerlegt werden müssen.

Es ist zu bedenken, dass bspw. bei einer Operation im Bauchraum vier, fünf oder sechs Trokare eingeschoben werden, um die verschiedenen Manipulationen bei einem Eingriff durchführen zu können, wobei die Trokare dann möglichst rasch nach einem Eingriff wieder für einen neuen Eingriff zur Verfügung stehen sollen.

Es ist daher Aufgabe der vorliegenden Erfindung ein Zugangsinstrument zu schaffen, das einfach aufgebaut und handhabbar ist und das sicher verankerbar ist.

Erfindungsgemäß wird die Aufgabe durch ein medizinisches Instrument zum Schaffen eines Zugangs für einen minimalinvasiven Eingriff dadurch gelöst, dass die Kantenflächen Verzahnungsmerkmale aufweisen, die ein seitliches Abgleiten der Teilkörper quer zu den Kantenflächen sperren.

Zum Einführen des Körpers in den menschlichen Körper können die einzelnen Teilkörper zunächst so zusammengesetzt oder zusammengelegt, dass diese zu einem distalen Körper zusammengefügt werden. Dieser Körper stellt einen etwa stabförmigen Körper dar.

In diesem Zustand kann nun das Instrument durch eine Inzision in den Körper eingeschoben werden, wobei die Einschubtiefe begrenzt ist, da in diesem Bauzustand die proximalen Teilkörperabschnitte seitlich abstehen. Die zusammengefügten distalen Teilkörperabschnitte fungieren in dieser Position als eine Art Trokarhülse.

Die distalen Teilkörperabschnitte können auch einzeln eingeschoben und im Körper zusammengefügt werden.

Das Gebilde, das einer Trokarhülse entspricht, besteht, über die Längsachse gesehen, aus zwei Teilkörpern, von denen wie bei einem "Y" oben die beiden proximalen Teilkörperabschnitte seitlich abstehen. Das Einführen in den Körper kann dabei klassisch unter Zuhilfenahme eines Trokardornes oder eines speziellen Eintreibers geschehen.

Nach dem Setzen werden die beiden seitlich abstehenden proximalen Teilkörperabschnitte in Richtung der Längsmittelachse aufeinander zubewegt, bis diese einen geschlossenen proximalen Hohlkörper bilden, der quasi dem Trokargehäuse entspricht.

Bei diesem Umklappen werden die bereits in den Körper eingeschobenen distalen Teilkörperabschnitte nach außen, von der Mittellängsachse radial gesehen, weg verschwenkt und bilden somit eine Sperre gegenüber einem Abziehen des Hohlkörpers von der Körperwand, also bspw. der Bauchdecke. Je nach Ausgestaltung kann die Schwenkbewegung soweit erfolgen, dass sich die Außenseite der verschwenkten distalen Teilkörperabschnitt bis an die Innenseite der Bauchdecke legt. In dieser zweiten Position fungieren die distalen Teilkörperabschnitte als Abziehsperre. Die gespreizten distalen Teilkörperabschnitte erlauben ein schräges Einführen von Instrumenten oder auch das Einführen mehrerer Instrumente, ohne diese zu behindern.

Das Vorsehen von Verzahnungsmerkmalen, die ein seitliches Abgleiten der Teilkörper quer zu den Kantenflächen sperren, hat den erheblichen Vorteil, dass, nachdem man beispielsweise die beiden distalen Teilkörperabschnitte zu dem distalen Hohlkörper zusammengelegt hat, ein seitliches Abrutschen oder Verschieben dieser Teile untereinander durch diese Verzahnungsmerkmale gehindert ist.

Das erleichtert insbesondere die Handhabung des Zusammensetzens und die Handhabung des Instruments in dieser ersten Position, also in der die zum distalen Hohlkörper zusammengesetzten distalen Teilkörperabschnitte an den Körper angesetzt und dann durch eine Inzision in den Körper, beispielsweise durch die Bauchdecke hindurch, eingeschoben werden können. Das verhindert, dass beispielsweise bei diesem Einschieben sich die aneinanderliegenden Teilkörperabschnitte in seitlicher Richtung verschieben und voneinander lösen. Diese Verzahnungsmerkmale fördern auch die Abrollbewegung längs der Kantenflächen beim Umschwenken der Teilkörperabschnitte, um dadurch die proximalen Teilkörperabschnitte zusammenzufügen. Diese Verschwenkbewegung muss meist gegen den Widerstand des Hautbereiches durchgeführt werden, durch den die distalen Teilkörperabschnitte durchgeschoben wurden. Meist erweitern sich die Teilkörperabschnitte in diesem gekrümmten Übergangsbereich, so dass durch den Gegendruck der Hautpartien die Gefahr besteht, dass die Teilkörper sich voneinander lösen, was durch die nunmehr vorgesehenen Verzahnungsmerkmale gehindert wird.

Die nunmehr zusammengesetzten proximalen Teilkörperabschnitte ergeben zum einen im Bereich der Bauchdecke den durch die Bauchdecke hindurchgeführten Durchgangskanal, also die "Trokarhülse", außerhalb der Bauchdecke bilden diese Abschnitte dann das "Trokargehäuse". Durch die Verzahnungsmerkmale liegen die proximalen Teilkörperabschnitte über deren Längskantenflächen eng aneinander, so dass diese Fügenaht dicht ausgebildet werden kann. Daher kann es ausreichen, wenn diese Verzahnungsmerkmale nur im Bereich der proximalen Teilkörperabschnitte vorhanden sind.

Zum Verschwenken aus der ersten Position in diese zweite Position kann ein Werkzeug zu Hilfe genommen werden, das an die noch seitlich abgespreizten proximalen Teilkörperabschnitte angelegt wird, oder, das kann auch durch die Hand einer Person durchgeführt werden, indem diese bspw. beide Teile zwischen Zeigefinger und Daumen ergreift und diese in Richtung der Mittellängsachse zusammenführt. Das Werkzeug kann auch an den zusammengelegten distalen Teilkörperabschnitten angesetzt werden und diese spreizen.

Ausgestaltungen derartiger Werkzeuge sind in der parallelen Anmeldung der Anmelderin (amtliches Aktenzeichen DE 10 2009 014 527.3; Titel: Vorrichtung zum Spreizen eines Zugangsinstrumentes für einen minimalinvasiven Eingriff) näher beschrieben.

Wie gesagt, gibt der Abschnitt, der in der Bauchdecke steckt, das Maß bzw. das Lumen vor, das dann für den minimalinvasiven Eingriff von der Außenseite her zur Verfügung steht.

Durch entsprechende Formgebung des gekrümmten Übergangsbereiches zwischen distalem Teilkörperabschnitt und proximalen Teilkörperabschnitt ergibt sich die Möglichkeit, diese Öffnung beim Verschwenken noch etwas zu dehnen, so dass ein relativ durchmessergroßes Lumen zur Verfügung steht, das erheblich größer sein kann als ein Lumen am distalen Ende des distalen Körpers. Das eröffnet die Möglichkeit, zunächst eine relativ kleine Inzision in der Haut einzubringen, durch die die zusammengefügten distalen Teilkörperabschnitte hindurchgeschoben werden, und anschließend ein sanftes Erweitern der Öffnung beim Verschwenken der proximalen Teilkörperabschnitte in Richtung der Mittellängsachse des Hohlkörpers zu bewerkstelligen.

Letztendlich resultiert ein extrem sicherer fester Sitz des Zugangsinstrumentes im menschlichen Körper, der ein axiales Verschieben, insbesondere ein Abziehen hindert dennoch gewisse Kippbewegungen ermöglicht.

In einer weiteren Ausgestaltung der Erfindung weisen die Kantenflächen aneinanderliegender Teilkörperabschnitte eine Nut- und Feder-Struktur auf.

Diese Maßnahme hat den Vorteil, dass die Nut- und Feder-Struktur nicht nur das seitliche Verschieben sperrt, sondern, dass beim Verschwenken der Teilkörperabschnitte diese Nut- und Feder-Struktur zugleich eine exakte Führung der Abrollbewegung beim Verschwenken der Teilkörperabschnitte zwischen der ersten und der zweiten Position, oder umgekehrt, bewirkt. Durch die Übergreifung im Nut/Feder-Bereich kann eine besonders gasdichte Fügenaht der aneinanderliegenden proximalen Teilkörperabschnitte geschaffen werden.

In einer weiteren Ausgestaltung der Erfindung weisen die Kantenflächen Vorsprünge und Vertiefungen auf, die mit entsprechenden Vertiefungen und Vorsprüngen an den Kantenflächen des anliegenden Teilkörperabschnittes in Eingriff treten.

Diese Maßnahme hat den Vorteil, dass durch konstruktiv einfache und einfach herzustellende Maßnahmen, das Halten der aneinandergefügten Teilkörperabschnitte gesichert wird. Man kann bspw. den Außendurchmesser der Vorsprünge etwas größer, oder in besonderer Form herstellen, die dann beim Aneinanderfügen in die entsprechenden Bohrungen oder Vertiefungen, richtiggehend hineingedrückt werden, so dass in diesem zusammengelegten Zustand, bspw. in der ersten Position, die Teilkörper nicht mehr voneinander abfallen können. Bei dieser Ausgestaltung resultiert auch ein Erschweren eines Lösens der aneinandergefügten Teilkörperabschnitte entgegen der Richtung, in der diese zusammengefügt wurden. In Kombination mit der Nut- und Feder-Konstruktion kann insbesondere im Übergangsbereich zwischen distalem und proximalem Teilkörperabschnitt eine sichere Halterung, eine gezielte Führung und eine gasdichte Abdichtung der aneinanderliegenden Kanten der Teilkörper verwirklicht werden.

In einer weiteren Ausgestaltung der Erfindung bilden die zusammengefügten distalen Teilkörperabschnitte einen stabartigen distalen Körper, dessen Durchmesser geringer ist, als der aus den zusammengesetzten proximalen Teilkörperabschnitten resultierende proximale Hohlkörper.

Diese Maßnahme hat den erheblichen Vorteil, dass zum Einführen zunächst ein relativ durchmessergeringer distaler stabartiger Körper zur Verfügung steht. Der eigentliche nach dem Verschwenken geschaffene Zugang wird dann durch die Konstruktion und Geometrie der zusammengefügten proximalen Teilkörperabschnitte bestimmt, wobei hier dann bspw. die zuvor erwähnte Aufweitung des Zugangkanales im Körper erfolgen kann. Gleichzeitig eröffnet der größere Durchmesser die Möglichkeit in diesem Bereich nun weitere Bauteile wie Klappen, Anschlüsse oder dergleichen vorzusehen. Es ist auch die Möglichkeit eröffnet, nunmehr mehrere Instrumente gleichzeitig, leicht zueinander verkippt, hindurchzuführen.

In einer weiteren Ausgestaltung der Erfindung weitet sich der stabartige distale Körper von distal nach proximal auf.

Diese Maßnahme hat den zusätzlichen Vorteil, dass eine extrem kleine Inzision von wenigen Millimetern ausreichen kann, an die das verjüngte distale Ende des Körpers angesetzt werden kann. Schon beim Einschieben des sich von distal nach proximal aufweiteten Körpers kann dabei die Öffnung etwas aufgeweitet werden. Dies hat sich bspw. bei Eingriffen im Nabelbereich als sehr günstig erwiesen.

In einer weiteren Ausgestaltung der Erfindung sind die zusammengefügten proximalen Teilkörperabschnitte mit einem nach proximal abschließenden Deckel versehen.

Das Vorsehen des Deckels hat den Vorteil, dass die proximale Seite der aneinandergefügten proximalen Teilkörperabschnitte abgeschlossen ist. Dieser Abschluss kann durch entsprechende Ausformung des proximalen Endes der proximalen Teilkörperabschnitte bewerkstelligt werden oder ein solcher Abschluss kann eingesetzt oder eingeschraubt werden.

In einer weiteren Ausgestaltung der Erfindung ist der Deckel als auf einem proximalen Rand des proximalen Hohlkörpers aufgesetzte Kappe ausgebildet.

Das Aufsetzen der Kappe kann beispielsweise die zuvor erwähnte Haltesicherung der beiden zusammengelegten oder zusammengeschwenkten proximalen Teilkörperabschnitte bewirken. Durch die Kappe ist es nunmehr auch möglich, den proximalen Hohlkörper proximalseitig gasdicht abzuschließen, so dass beispielsweise dann durch diesen abgeschlossenen proximalen Hohlkörper Gase zum Aufblähen des inneren Hohlraums zugeführt werden können.

In einer weiteren Ausgestaltung der Erfindung weist die Kappe eine Dichtung auf, die den proximalen Hohlkörper nach proximal abdichtet.

Diese Dichtung kann dabei mehrere Dichtungsöffnungen aufweisen.

Diese Dichtung kann mit entsprechenden Schlitzen oder sonstigen Ausformungen versehen sein, so dass, wenn gewünscht, durch die Dichtung hindurch schaftartige Instrumente eingeschoben werden können, die dann wiederum durch die Dichtung zur Außenseite hin abgedichtet werden. Durch entsprechende Ausgestaltung der Dichtung bzw. der Kappe können dann je nach Eingriff unterschiedliche Typen von Dichtungen eingesetzt werden, die das Einschieben von mehreren oder unterschiedlichen großen Instrumenten ermöglichen. Auch hier sind nun sehr zahlreiche Variationsmöglichkeiten eröffnet.

Nach dem minimalinvasiven Eingriff wird zunächst die Kappe abgenommen und dann werden die zusammengefügten proximalen Teilkörperabschnitte wieder von der Mittellängsachse wegbewegt, wobei dann die im Innern des Körpers gelegenen distalen Teilkörperabschnitte wieder zu dem distalen Körper zusammengelegt werden, der dann über die Inzision vom Körper abgezogen werden kann. Auch hier können die Teilkörper auch wieder einzeln vom Körper abgezogen werden.

Die Querschnittsgeometrie dieses distalen Körpers kann zylindrisch, oval, eckig, dreieckig oder sonstwie sein, solange es einen schlanken Körper ergibt, der durch eine Inzision in den Körper eingeschoben werden kann. Dieser Körper kann massiv oder als Hohlkörper ausgebildet sein.

Ein besonderer Vorteil dieser Ausgestaltung besteht somit darin, dass keine zusätzlichen Bauelemente vorgesehen werden müssen, die von der Trokarhülse weggespreizt werden, denn die Trokarhülse selbst wird aufgespreizt. Ein weiterer Vorteil besteht darin, dass für diese Vorgänge keine zusätzliche Mechanik in den Hohlkörper eingebaut werden muss, sondern das Verschwenken der abgewinkelten Teilkörperabschnitte kann von der Außenseite her erfolgen.

Nach Abziehen vom Körper kann dann der Körper des Zugangsinstruments in die einzelnen Teilkörper zerlegt und einfach gereinigt und sterilisiert werden.

Zuvor wurde das anhand eines Körpers beschrieben, der aus zwei halbschalenartigen Teilkörpern zusammengesetzt wird, es ist aber einleuchtend, dass dieser Körper auch aus drei, vier oder mehr Teilkörpern zusammensetzbar ist, das Grundprinzip bleibt immer gleich.

Somit wird auf konstruktiv und mechanisch einfache Art und Weise ein stabiler, fest sitzender unverrückbarer Zugang für einen minimalinvasiven Eingriff geschaffen, wobei das mit wenigen Bauteilen erfolgt, die robust sind, einfach zu handhaben und auch einfach gereinigt und sterilisiert werden können.

In einer weiteren Ausgestaltung der Erfindung sind an der Außenseite des distalen Teilkörperabschnittes Außengewindeabschnitte angeformt.

Diese Maßnahme hat den Vorteil, dass diese Außengewindeabschnitte ein Eindrehen des zusammengelegten distalen Körpers erleichtern oder fördern. Das Einbringen eines Trokars in den Körper ist eine unblutige Sache, das ist gerade der Vorteil der minimalinvasiven Chirurgie. Das heißt, die Haut wird nur im Oberflächenbereich angeschnitten und der hülsenförmige Körper muss lediglich durch die subkutanen Schichten und eine möglicherweise darunterliegende Fettschicht hindurchgetrieben werden, um den inneren Hohlraum zu erreichen. Die Maßnahme mit den Gewindeabschnitten eröffnet nun die Möglichkeit, dies nicht mit einer nur linear gerichteten Bewegung durchzuführen, sondern mit einer sanften geführten Drehbewegung, was in einem besonders atraumatischen Einführen in den Körper resultiert.

In einer weiteren Ausgestaltung der Erfindung erfolgt der Übergang von der ersten Position in die zweite Position, und umgekehrt, durch ein Verschwenken der Teilkörperabschnitte, wobei sich jeweils die zusammengefügten Teilkörperabschnitte voneinander wegbewegen und die vormals nach außen abstehenden Teilkörperabschnitte zu einem Körper zusammengefügt werden.

Dieser "synchrone" Übergang erlaubt eine besonders schonende Handhabung des medizinischen Instruments, denn je weiter bspw. die proximal vorstehenden, vormals abstehenden Abschnitte aufeinander zubewegt werden, desto weiter sind bereits die im Körper eingeschobenen distalen Teilkörperabschnitte schon voneinander gespreizt. Es sind Zwischenpositionen vorhanden, in denen die proximal vorstehenden Teilkörperabschnitte noch nicht vollständig zusammengelegt sind und demzufolge die distalen Teilkörperabschnitte noch nicht vollständig gespreizt sind. In diesem Zustand sind noch geringe Korrekturen am Sitz von der Person möglich, die gerade diese Verschwenkbewegung durchführt. Soll bspw. durch die Ausformung der proximalen Teilkörperabschnitte eine Aufweitung der Öffnung erfolgen, kann die Handhabungsperson in einer solchen Zwischenposition den genauen Sitz kontrollieren, d.h. ob auch genau die Abschnitte, die zum Spreizen vorgesehen sind, auch an der richtigen Stelle liegen. Dies erleichtert die Handhabung und führt zu einem exakt gewollten Sitz der Vorrichtung nach dem Spreizen der distalen Teilkörperabschnitte.

In einer weiteren Ausgestaltung der Erfindung erfolgt die Verschwenkbewegung um eine Schwenkachse im Übergangsbereich vom distalen zum seitlich abstehenden proximalen Teilkörperabschnitt.

Diese Schwenkachse, die eine rein virtuelle Schwenkachse sein kann, erleichtert die Verschwenkbewegung und trägt zu einer harmonischen Schwenkbewegung bei, die zielgerichtet um die Schwenkachse erfolgt.

Das steht auch im Zusammenhang mit dem zuvor erwähnten Vorteil, dass man die Schwenkachse auch an bestimmte Stellen bringen kann, die bspw. einer zusätzlichen Aufweitung förderlich sind. Der Hauptvorteil liegt aber in einer sicher geführten Schwenkbewegung.

In einer weiteren Ausgestaltung der Erfindung sind die Teilkörper über Drehgelenke aneinander gehalten, um deren Schwenkachse die Schwenkbewegung erfolgt.

Diese Maßnahme hat den Vorteil, dass die Teilkörper über die Drehgelenke zu einem Gesamtkörper verbunden sind. Die Drehgelenke selbst bilden dann ganz klar definierte Schwenkachsen, um die die jeweiligen proximalen bzw. distalen Teilkörperabschnitte eines Teilkörpers verschwenkt werden. Bei einer Konstruktion mit bspw. zwei Teilkörperabschnitten würden zwei, diametral zur Längsachse gesehen, gegenüberliegende Drehgelenke resultieren, über die die beiden Teilkörper verbunden sind und verschwenkt werden können.

In einer weiteren Ausgestaltung der Erfindung weisen die Teilkörper im Bereich der Schwenkachse komplementäre Scharniergelenkmerkmale auf, die die Schwenkbewegung führen, ohne dass die Teilkörper fest miteinander verbunden sind.

Diese Maßnahme hat nun den erheblichen Vorteil, dass durch die Scharniergelenkmerkmale bei aneinandergelegten Teilkörpern eine exakt geführte Verschwenkbewegung erfolgt, wie das auch bei der zuvor erwähnten Ausgestaltung mit den Drehgelenken der Fall ist, gleichzeitig besteht die Möglichkeit, diese beiden Teilkörper nach Gebrauch voneinander zu trennen und zum Reinigen und zum Sterilisieren einzeln zu behandeln. Solche Scharniergelenkmerkmale sind bspw. eine pfannenartige Ausbuchtung in dem einen Körper, in dem ein Kopf, der vom anderen Teilkörper hervorspringt, eingelegt werden kann.

In einer weiteren Ausgestaltung der Erfindung springt vom distalen Ende eines distalen Körperabschnittes eine nach außen gerichtete Nase vor.

Diese Maßnahme hat den Vorteil, dass diese Nasen hilfreich beim Einführen der Teilkörperabschnitte sind. Sollen bspw. die beiden Teilkörperabschnitte einzeln in den Körper eingeführt werden, kann ein solcher Teilkörper mit seiner Nase an der Inzision im Körper, bspw. an der Bauchdecke, angesetzt und durch die Inzision eingeschoben werden. Diese Nasen sind hilfreich dabei, die betroffenen Gewebeschichten vollständig zur Seite zu drängen, so dass der Einführvorgang der distalen Teilkörperabschnitte in den Körper erleichtert ist.

In einer weiteren Ausgestaltung der Erfindung beträgt ein Spreizwinkel α zwischen den distalen Teilkörperabschnitten bei zusammengefügten proximalen Teilkörperabschnitten mehr als 90°.

Diese Maßnahme hat den Vorteil, dass Instrumente auch schräg in den Körper eingeführt werden können und nicht durch die aufgespreizten distalen Teilkörperabschnitte behindert werden.

Insbesondere wenn mehrere Instrumente durch den Hohlkörper gleichzeitig hindurchgeschoben werden sollen, was gerade ein besonderer Vorteil der erfindungsgemäßen Vorrichtung ist, ist dieses weite Aufspreizen der distalen Teilkörperabschnitte sehr hilfreich. Dies unterstützt den Hauptzweck, nämlich mit einem einzigen Zugang in den Körper (Single Port Access) mehrere Instrumente gleichzeitig einführen zu können.

In einer weiteren Ausgestaltung der Erfindung beträgt ein Winkel β zwischen einer äußeren Längskante eines proximalen Teilkörperabschnittes und des entsprechenden distalen Körperabschnittes, in einer Verschwenkebene gesehen, mehr als 90°.

Diese Maßnahme hat den Vorteil, dass beim Einführen eines Teilkörperabschnittes in den Körper durch die Inzision hindurch der Handhabungsperson ausreichend Raum zur Verfügung steht, um den proximalseitigen Teilkörperabschnitt rundum von Hand zu ergreifen.

Insbesondere in der Ausgestaltung mit der vorspringenden Nase ist diese Konstruktion vorteilhaft, denn dann kann in einer zügigen und zielgerichteten Bewegung ein Teilkörper angesetzt und eingeschoben werden, indem dieser mit der Hand am proximalen Teilkörperabschnitt rundum ergriffen wird.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Ansicht eines Teilkörperabschnittes einer ersten Ausführungsform, die aus zwei halbschalenförmigen Teilkörperabschnitten besteht;
- Fig. 2: eine Seitenansicht dieser Ausführungsform, bei der zwei distale Teilkörperabschnitte zu einem distalen rohrförmigen Hohlkörper zusammengesetzt sind und die proximalen Teilkörperabschnitt, Y6artig seitlich abstehen;
- Fig. 3: eine Zwischenposition bei einer Verschwenkbewegung der beiden Teilkörperabschnitte umeinander bzw. gegeneinander;
- Fig. 4: eine Seitenansicht der beiden Teilkörperabschnitte in einer zweiten Position nach Verschwenken aus der in Fig. 2 dargestellten ersten Position, über die in Fig. 3 dargestellte Zwischenposition;
- Fig. 5: eine Draufsicht auf den Zusammenbau von Fig. 4 von proximal;
- Fig. 6: eine der Seitenansicht von Fig. 4 entsprechende Ansicht, wobei auf die zusammengefügten proximalen Teilkörperabschnitte eine Kappe aufgesetzt ist;
- Fig. 7: einen Längsschnitt durch den Zusammenbau von Fig. 6, wobei zusätzlich angedeutet ist, wie dieser Zusammenbau in einer Bauchdecke aufgenommen ist;
- Fig. 8: eine Ansicht des Zusammenbaus von Fig. 6 von distal;
- Fig. 9: eine perspektivische Darstellung eines zweiten Ausführungsbeispieles, wobei diese Position der in Fig. 2 dargestellten ersten Position des ersten Ausführungsbeispieles entspricht;
- Fig. 10: eine der Darstellung von Fig. 4 entsprechende perspektivische Darstellung des zweiten Ausführungsbeispiels nach dem Verschwenken von der ersten in die zweite Position;
- Fig. 11: eine der Fig. 2 vergleichbare Darstellung eines dritten Ausführungsbeispieles deren Kanten Scharniergelenkmerkmale aufweisen;
- Fig. 12: eine der Darstellung von Fig. 3 vergleichbare Zwischenposition;
- Fig. 13: eine der Fig. 4 vergleichbare Darstellung der zweiten Position dieses Ausführungsbeispiels;
- Fig. 14: eine teilweise vereinfachte perspektivische Darstellung der Zwischenposition von Fig. 12;
- Fig. 15: eine der perspektivischen Darstellung von Fig. 14 entsprechende Darstellung eines vierten Ausführungsbeispiels mit festen Gelenkachsen;
- Fig. 16: eine perspektivische Darstellung eines fünften Ausführungsbeispiels, das aus vier Teilkörperabschnitten zusammengesetzt ist, und zwar in der ersten Position;
- Fig. 17: eine perspektivische Darstellung des medizinischen Instruments von Fig. 16 in der zweiten Position mit seitlich abgespreizten distalen Teilkörperabschnitten;
- Fig. 18: eine perspektivische Darstellung eines sechsten Ausführungsbeispiels mit seitlich vorspringenden Nasen, mit aneinandergefügten distalen Teilkörperabschnitten;
- Fig. 19: eine im Bereich der distalen Teilkörper längs der Linie XIX-XIX in Fig. 18 geschnittene Seitenansicht des sechsten Ausführungsbeispieles;
- Fig. 20: eine Ansicht von distal nach proximal des sechsten Ausführungsbeispieles;
- Fig. 21: eine Seitenansicht des sechsten Ausführungsbeispieles und aufgesetzter Kappe in einer Bauchdecke steckend;
- Fig. 22: eine Seitenansicht des sechsten Ausführungsbeispieles mit Kappe, und
- Fig. 23: eine Draufsicht von distal nach proximal des sechsten Ausführungsbeispieles.

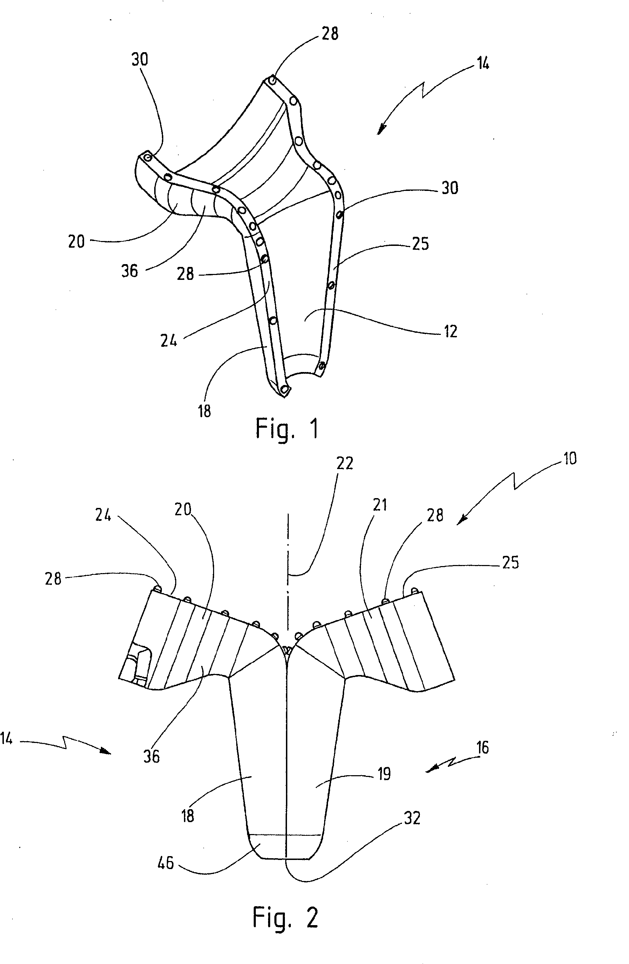

Ein in den Figuren 1 bis 8 dargestelltes erstes Ausführungsbeispiel eines medizinischen Instrumentes ist in seiner Gesamtheit mit der Bezugsziffer 10 bezeichnet.

Das medizinische Instrument 10 weist einen Körper auf, der als ein Hohlkörper 12 ausgebildet ist, wobei in Fig. 1 ein halbschalenförmiger Teilkörper 14 dieses Hohlkörpers dargestellt ist.

Wie das aus Fig. 2 zu erkennen ist, existiert noch ein Teilkörper 16, der identisch aufgebaut ist wie der Teilkörper 14.

Der Teilkörper 14 weist einen distalen Teilkörperabschnitt 18 auf, der einem halben, längs seiner Längsachse aufgeschnittenen, sich von distal nach proximal erweiternden Rohr entspricht.

Demzufolge weist der Teilkörper 16 einen entsprechenden distalen Teilkörperabschnitt 19 auf.

An den in Fig. 1 dargestellten distalen Teilkörperabschnitt 18 schließt sich ein proximaler Teilkörperabschnitt 20 an, der gegenüber dem distalen Teilkörperabschnitt 18 seitlich nach außen gebogen ist. Auch der proximale Teilkörperabschnitt 20 besteht in Form einer Halbschale. Demzufolge weist der distale Teilkörperabschnitt 19 einen entsprechenden proximalen Teilkörperabschnitt 21, wie das in Fig. 2 ersichtlich ist.

Jeder der Teilkörper 14 und 16 weist axial verlaufende Kantenflächen 24 und 25 auf. Wie aus der perspektivischen Darstellung von Fig. 1 ersichtlich ist, stehen von der Kantenfläche 24 im distalen Abschnitt Vorsprünge 28 vor, wohingegen im proximalen Abschnitt dieser Kantenfläche 24 Vertiefungen 30 vorhanden sind. An der benachbarten Kantenfläche 25 ist diese Anordnung genau umgekehrt, im distalen Abschnitt der Kantenfläche 25 sind Vertiefungen 30 vorhanden, im proximalen Abschnitt der Kantenfläche 25 entsprechende Vorsprünge 28. Wie bereits zuvor erwähnt, ist die Ausgestaltung der Kantenflächen am zweiten Teilkörper 16 gleich. Werden die beiden identischen Teilkörper 14 und 16 wie in Fig. 2 gezeigt zusammengesetzt, ist an der Stelle, an der beim Teilkörper 14 ein Vorsprung 28 vorsteht, in der entsprechenden Kantenfläche am Teilkörper 16 eine Vertiefung 30 vorhanden, in die ein solcher Vorsprung 28 eingeschoben werden kann und umgekehrt.

Werden nun die beiden Teilkörper 14 und 16 so aneinandergelegt, dass die distalen Teilkörperabschnitte 18 und 19 über deren Kantenflächen 24 und 25 aneinanderliegen, entsteht ein Körper, wie er aus Fig. 2 ersichtlich ist. Dabei sind die Vorsprünge 28, die von den Kantenflächen des einen Teilkörpers vorstehen in die entsprechenden Vertiefungen 30 der anderen Kantenflächen eingetreten. Letztendlich resultiert, wie das aus Fig. 2 ersichtlich ist, ein distaler Hohlkörper 32, der sich von distal nach proximal gesehen, konisch etwas aufweitet.

Die proximalen Teilkörperabschnitte 20 und 21 sind dabei von der Mittellängsachse 22 seitlich weg gerichtet oder gebogen.

Dies entspricht der ersten Position des medizinischen Instrumentes 10.

In dieser ersten Position kann der distale Hohlkörper 32 bspw. über eine Inzision durch eine Bauchdecke in einen Hohlraum, insbesondere den Bauchraum eingeschoben werden, wie das aus der Handhabung von Trokaren bekannt ist.

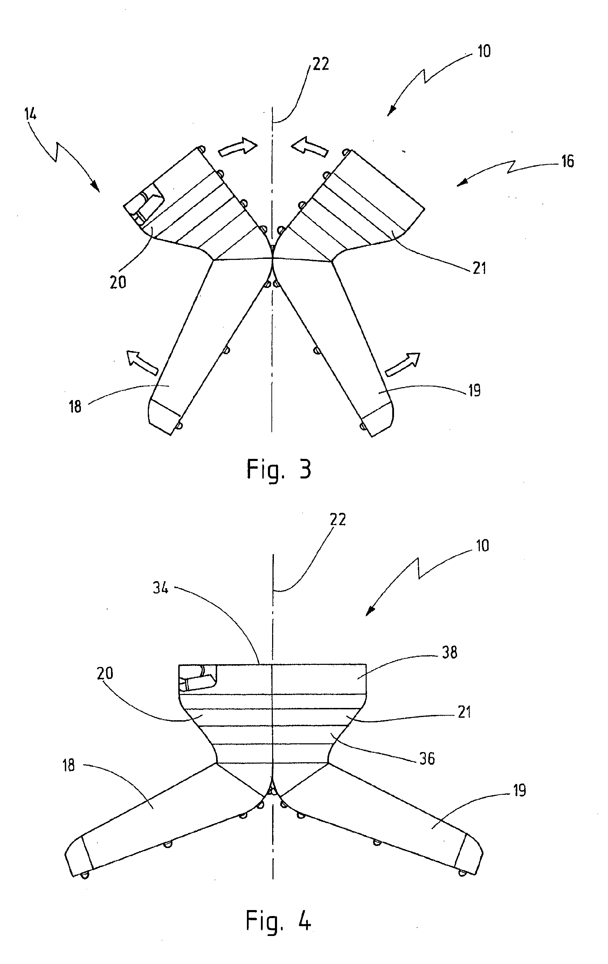

Der Zusammenbau, wie er aus Fig. 2 ersichtlich ist, kann nun in die in Fig. 4 gezeigte zweite Position verschwenkt oder umgeklappt werden, wobei die in Fig. 3 dargestellte Zwischenposition durchlaufen wird.

Dazu werden, wie dies in Fig. 3 durch Pfeile angedeutet ist, die beiden seitlich abstehenden proximalen Teilkörperabschnitte 20 und 21 in Richtung der Mittellängsachse 22 aufeinander zubewegt, wobei dies von Hand durch eine Handhabungsperson bewerkstelligt werden kann, indem bspw. an die jeweils gegenüberliegenden äußeren Ränder der Zeigefinger und der Daumen angelegt wird und diese Teile aufeinander zu verschwenkt werden. Gleichzeitig spreizen sich bei dieser Bewegung die distalen Teilkörperabschnitte 18 und 19 von der Mittellängsachse 22 weg, wie dies in Fig. 3 durch die Pfeile angedeutet ist. Es können dazu auch spezielle Werkzeuge herangezogen werden, wie sie in der eingangs erwähnten parallelen Anmeldung beschrieben sind.

Diese Schwenk- oder Klappbewegung kann soweit durchgeführt werden, bis die proximalen Teilkörperabschnitte 20 und 21 über deren Kantenflächen 24 und 25 aneinanderliegen, somit einen proximalen Hohlkörper 34 bilden.

Der Übergang von dem Zustand von Fig. 3 zur Fig. 4 läuft sehr harmonisch ab, da die beiden Teilkörper 14 und 16 dabei über ihre Längskantenflächen 24 und 25 aneinander abrollen. Die Vorsprünge 28 und die Vertiefungen 30 stellen Verzahnungsmerkmale dar, die ein seitliches Abgleiten der Teilkörper 14 und 16, quer zu den Längskantenflächen 24 und 25 gesehen, sperren.

Aus der Darstellung von Fig. 4 ist ersichtlich, dass die proximalen Teilkörperabschnitte 20 und 21 über einen aufgeweiteten Bereich 36 in einen ringflanschartigen Endbereich 38 mit wesentlich größerem Durchmesser übergehen.

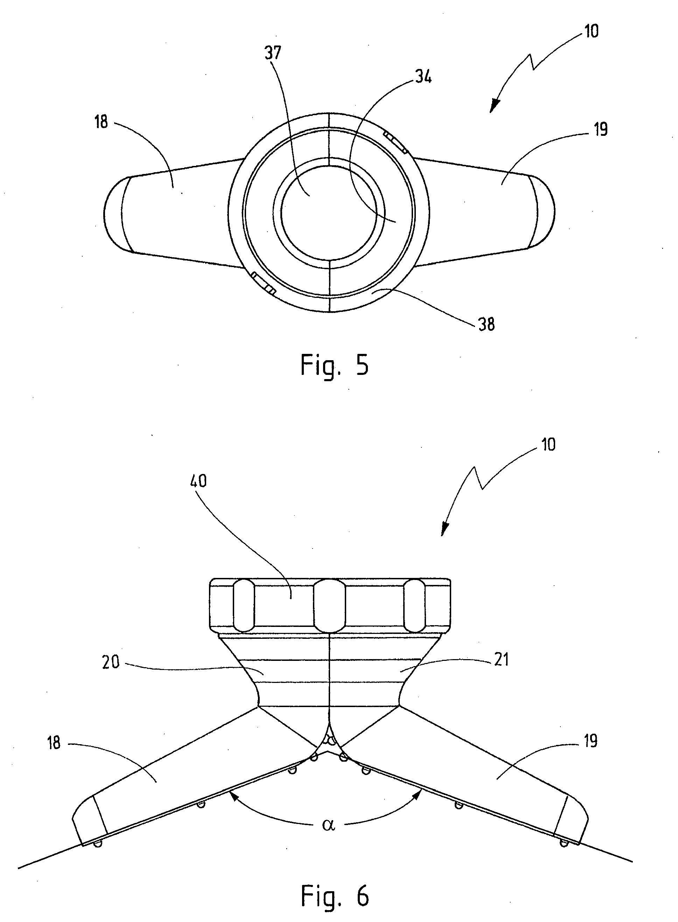

Daraus resultiert dann der in Fig. 4 und Fig. 5 ersichtliche sich nach proximal aufweitende zunächst nach oben offene proximale Hohlkörper 34. Aus der Draufsicht von Fig. 5 ist ersichtlich, dass eine Zugangsöffnung 37 geschaffen wurde, deren Durchmesser größer ist, als der Durchmesser des distalen Endes der zusammengelegten distalen Teilkörperabschnitte, wie das aus Fig. 2 ersichtlich ist.

Somit ist durch diese Bewegung auch eine Spreizung oder Aufweitung der Öffnung im Körper erfolgt, die nach Einschieben des distalen Hohlkörpers 32 resultiert. Eine Fase 46 am distalen Endbereich der distalen Teilkörperabschnitte 18 und 19 erleichtert das Einführen in oder durch die Inzision.

Aus den Darstellungen von Fig. 4 und Fig. 5 ist außerdem ersichtlich, dass ein relativ großvolumiger aufgeweiteter proximaler Hohlkörper 34 entstanden ist, der aus den zwei halbschalenförmigen proximalen Teilkörperabschnitten 20 und 21 zusammengesetzt ist.

In Fig. 6 ist dargestellt, dass eine Kappe 40 auf das proximale Ende dieses proximalen Hohlkörpers 34 aufgesetzt ist, wobei diese aufgeschraubt, aufgerastet oder in einer Art Bajonettverschluss aufgesetzt werden kann. In Fig. 6 ist zu erkennen, dass ein Spreizwinkel α zwischen den distalen Teilkörperabschnitten 18 und 19 bei aneinandergefügten proximalen Teilkörperabschnitten 20 und 21 größer als 90° ist, hier etwa 150° beträgt. Dadurch können Instrumente auch schräg ein- bzw. durchgeführt werden.

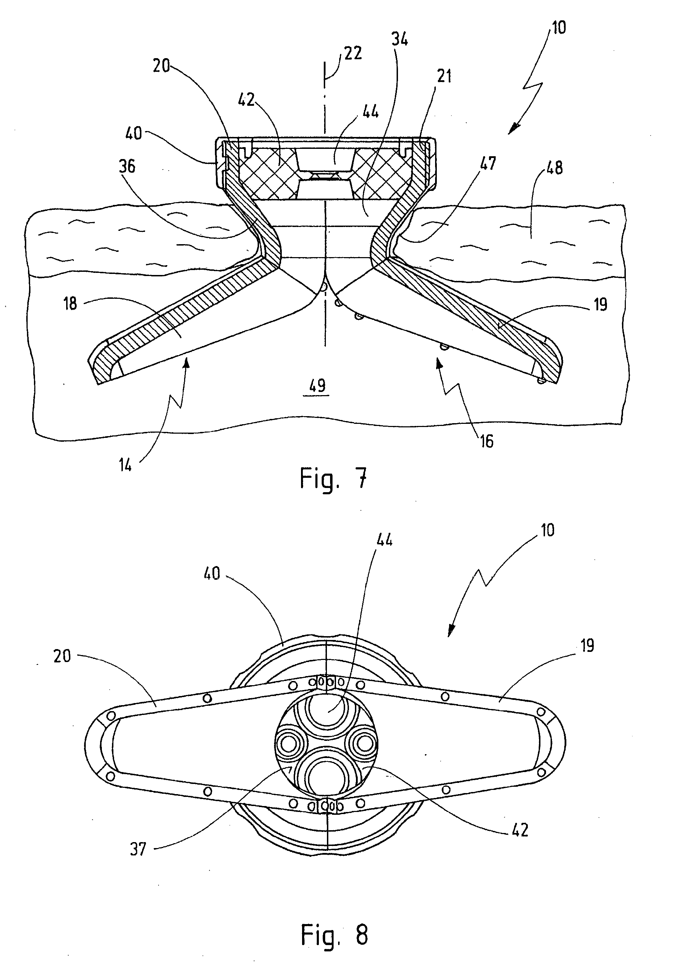

Aus der Schnittdarstellung von Fig. 7 und aus Fig. 8 ist zu entnehmen, dass in der Kappe 40 eine Dichtung 42 eingesetzt ist, in der zumindest eine Öffnung 44 vorgesehen ist, durch die ein Instrument in dichtender Weise durch das medizinische Instrument 10 hindurchgeschoben werden kann.

In Fig. 7 ist dargestellt, wie das medizinische Instrument 10 in eine Bauchdecke 48 eines menschlichen Körpers eingesetzt ist, um einen Zugang zu einem Hohlraum 49 unterhalb der Bauchdecke 48 zu schaffen.

Wie bereits zuvor erwähnt, wurde zunächst eine Inzision, sprich ein kleiner Einschnitt, in die Haut durchgeführt, die bspw. dem Durchmesser des distalen Endes des distalen Hohlkörpers 32 entspricht, wie das aus Fig. 2 ersichtlich ist. Die Fase 46 erleichtert dann ein Ansetzen oder Einschieben durch diese Inzision.

Gegebenenfalls unter Zuhilfenahme eines an sich bekannten Trokardornes, kann nun das medizinische Instrument 10, wie es in Fig. 2 dargestellt, durch die in Fig. 7 dargestellte Bauchdecke hindurchgeschoben werden. Nach dem Spreizen der distalen Teilkörperabschnitte 18 und 19 liegen diese zumindest teilweise an der Unterseite der Bauchdecke 48 an und sorgen somit für einen festen Sitz des medizinischen Instrumentes 10, und zwar sowohl gegen übermäßiges Verkippen als auch gegen Abziehen oder Einschieben. Der zuvor erwähnte Spreizwinkel α im Bereich von 150° hindert nicht nur ein Verkippen, sondern erlaubt, mehrere schaftartige Instrumente gleichzeitig auch in schrägen Richtungen durchzuführen.

Der aufgeweitete Bereich 36 der proximalen Teilkörperabschnitte 20 und 21 sorgt für eine Begrenzung der Einschubtiefe der proximalen Teilkörperabschnitte 20 und 21 durch die Bauchdecke 48 und sorgt auch zugleich für die zuvor beschriebene Spreizung der Öffnung 47 in der Bauchdecke 48.

Aus Fig. 7 ist deutlich, dass in diesem Zustand, also in der zweiten Position, das medizinische Instrument 10 unverrückbar, d.h. axial unverschiebbar und nicht kippgefährdet, an der Bauchdecke 48 sitzt. Nunmehr können durch das so gesetzte medizinische Instrument 10 entsprechende chirurgische Eingriffe vorgenommen werden.

Nach dem Eingriff wird die Kappe 40 abgenommen und die beiden proximalen Teilkörperabschnitte 20 und 21 werden, von der Mittellängsachse 22 ausgesehen, seitlich verschwenkt, durchlaufen also die Folge von Fig. 4 zu Fig. 3 bis zu Fig. 2.

In diesem Zustand kann das medizinische Instrument 10 dann wieder einfach von der Bauchdecke 48 abgezogen werden.

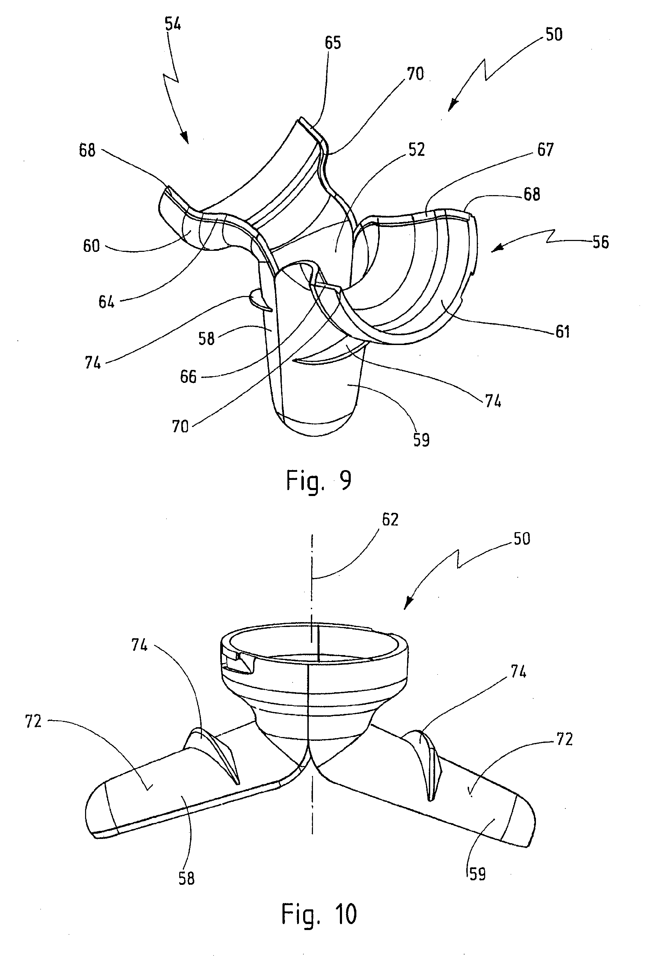

Bei dem in den Figuren 9 und 10 dargestellten zweiten Ausführungsbeispiel eines medizinischen Instrumentes entsprechend der Erfindung, ist dieses in seiner Gesamtheit mit der Bezugsziffer 50 bezeichnet.

Auch das medizinische Instrument 50 ist als ein Hohlkörper 52 ausgebildet, der aus zwei identischen Teilkörpern 54 und 56 zusammengesetzt ist.

Auch hier weist jeder Teilkörper 54 bzw. 56 wieder einen distalen Teilkörperabschnitt 58 bzw. 59 und einen seitlich abstehenden proximalen Teilkörperabschnitt 60 bzw. 61 auf. Die in Fig. 9 dargestellte Position entspricht somit der in Fig. 2 dargestellten ersten Position des ersten Ausführungsbeispieles.

Entlang der axial verlaufenden Kantenflächen 64 und 66 ist eine Nut- und Feder-Konstruktion vorhanden.

Eine axial verlaufende Kantenfläche 64 des Teilkörpers 54 ist in deren proximalen Bereich mit einer Feder 68 versehen. Die andere Kantenfläche 65 ist mit einer Nut 70 versehen.

Die axial verlaufenden Kantenflächen 67 und 66 des anderen Teilkörpers 56 sind ebenso ausgebildet. Das heißt, beide Teilkörper 54 und 56 sind identisch ausgebildet. Daher steht in Fig. 9 der Nut 70 des Teilkörpers 54 eine Feder 68 des Teilkörpers 56 gegenüber. Entsprechendes gilt für die benachbarten Kantenflächen. Diese Nut- und Feder-Konstruktion kann sich bis in die distalen Teilkörperabschnitte 58 und 59 hinein erstrecken.

Diese Nut- und Feder-Konstruktion sorgt nicht nur dafür, dass die beiden, wie in Fig. 9 zusammengefügten Teilkörper 54 und 56 sich nicht seitlich voneinander lösen, sondern auch, dass bei der Verschwenkbewegung von der ersten Position, wie sie in Fig. 9 dargestellt ist, zu der zweiten Position, wie sie in Fig. 10 dargestellt ist, eine Führung durch die Nut- und Feder-Konstruktion erfolgt. Wie aus Fig. 10 ersichtlich, resultiert in der zweiten Position wieder ein Körper, wie im Beispiel zuvor anhand der Fig. 4 beschrieben worden ist. Die Nut- und Feder-Konstruktion erlaubt eine gasdichte Aneinanderfügung der proximalen Teilkörper 54 und 56 längs deren Kantenflächen.

An den Außenseiten 72 der distalen Teilkörperabschnitte 58 und 59 sind Außengewindeabschnitte 74 angebracht, die das Einschieben der medizinischen Instrumentes 50 in der in Fig. 9 dargestellten Position in eine Öffnung in einer Bauchdecke in Form einer Drehbewegung begünstigen und führen. Das Gleiche gilt dann beim Herausdrehen der zusammengelegten distalen Teilkörperabschnitte 58 und 59 aus dem Körper nach einem Eingriff. Auch hier sind wieder zu der Mittellängsachse 62 symmetrische Hohlkörper 52 vorhanden, die hier ebenfalls aus zwei Teilkörpem 54 und 56 zusammengesetzt sind.

Auch hier ist wieder eine Kappe aufgesetzt.

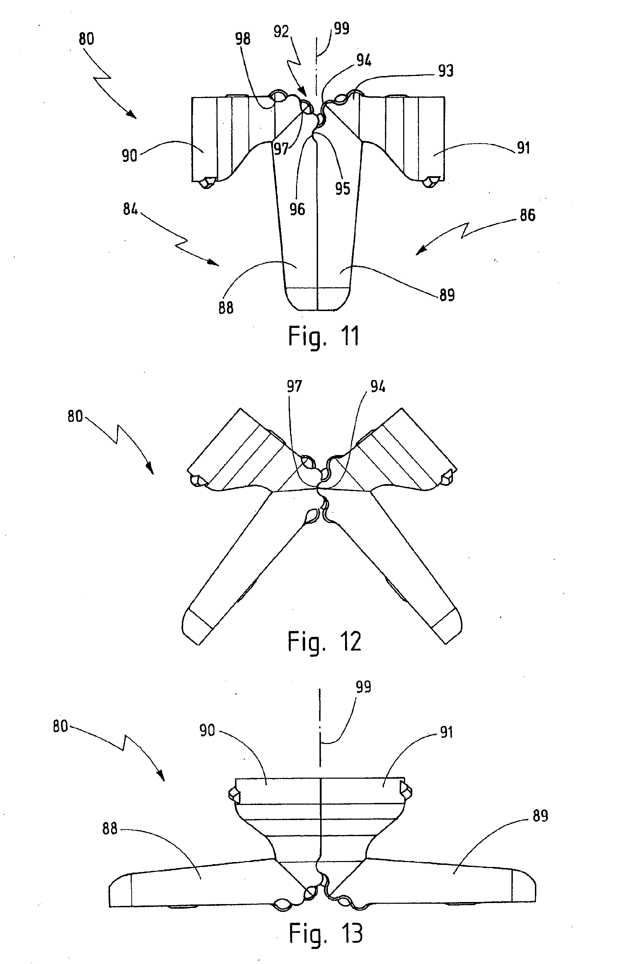

Bei einem dritten in den Figuren 11 bis 14 dargestellten Ausführungsbeispiel eines medizinischen Instrumentes, ist dies in seiner Gesamtheit mit der Bezugsziffer 80 bezeichnet.

Aus den Figuren 11 bis 14 ist zu entnehmen, dass auch hier wieder ein Hohlkörper 82 vorliegt, der aus zwei identischen halbschalenförmigen Teilkörpern 84 und 86 zusammengesetzt ist. Auch hier sind wieder distale Teilkörperabschnitte 88 und 89 vorhanden, die entsprechend, wie zuvor beschrieben, zusammengesetzt werden können, wie das in Fig. 11 dargestellt ist, wobei die proximalen Teilkörperabschnitte 90 und 91, hier etwa rechtwinklig seitlich, von der Mittellängsachse 99 abstehen bzw. verschwenkt sind.

Auch hier kann, wie das durch den Übergang von Fig. 11 über Fig. 12 zur Fig. 13 dargestellt ist, dieser Zusammenbau verschwenkt werden, so dass die proximalen Teilkörperabschnitte 90 und 91 einen proximalen Hohlkörper bilden.

Im Übergangsbereich zwischen dem jeweiligen distalen Teilkörperabschnitt zum jeweiligen proximalen Teilkörperabschnitt sind Scharniergelenke 92 angeordnet. Diese bestehen aus Vorsprüngen und Vertiefungen an den Längskantenflächen.

Wie das in Fig. 11 auf der rechten Seite erläutert ist, sind auf der dem Betrachter zugewandten Längskantenfläche des Teilkörpers 86 drei scheibenartige Gelenkköpfe 93, 94 und 95 vorgesehen, die in entsprechende komplementäre Gelenkpfannen 96, 97 und 98 in dem Teilkörper 84 eintreten können.

Dabei sind auch an der dem Betrachter zugewandten Kantenfläche des Teilkörpers 84 entsprechende, hier nicht näher bezeichnete, Gelenkköpfe vorhanden, die in Gelenkpfannen am Teilkörper 86 eingreifen, die zwischen dessen Gelenkköpfen 93, 94 und 95 vorhanden sind. An den jeweils hinteren Kantenflächen sind entsprechend komplementäre Anordnungen vorhanden, nur umgekehrt. Somit sind die beiden Teilkörper 84 und 86 wieder identisch aufgebaut.

Aus dem Übergang von Fig. 11 zu Fig. 13 und auch insbesondere an der Zwischenposition von Fig. 12 ist zu erkennen, dass durch diese Scharniergelenke 92 ein sanftes zielgerichtetes Abrollen der beiden Teilkörper 84 und 86 bei der Verschwenkbewegung stattfindet. Die Einsatzweise ist gleich wie bei dem zuvor beschriebenen Ausführungsbeispielen, es kann auch hier wieder eine Kappe aufgesetzt werden. Aus Fig. 13 ist ersichtlich, dass hier die distalen Teilkörperabschnitte 88 und 89 unter einem Spreizwinkel α von 180° stehen, so dass diese über einen langen und großflächigen Bereich an der Unterseite der Bauchdecke anliegen können und somit für einen besonders sicheren Halt sorgen. Auch hier wird eine (hier nicht dargestellte) Kappe aufgesetzt.

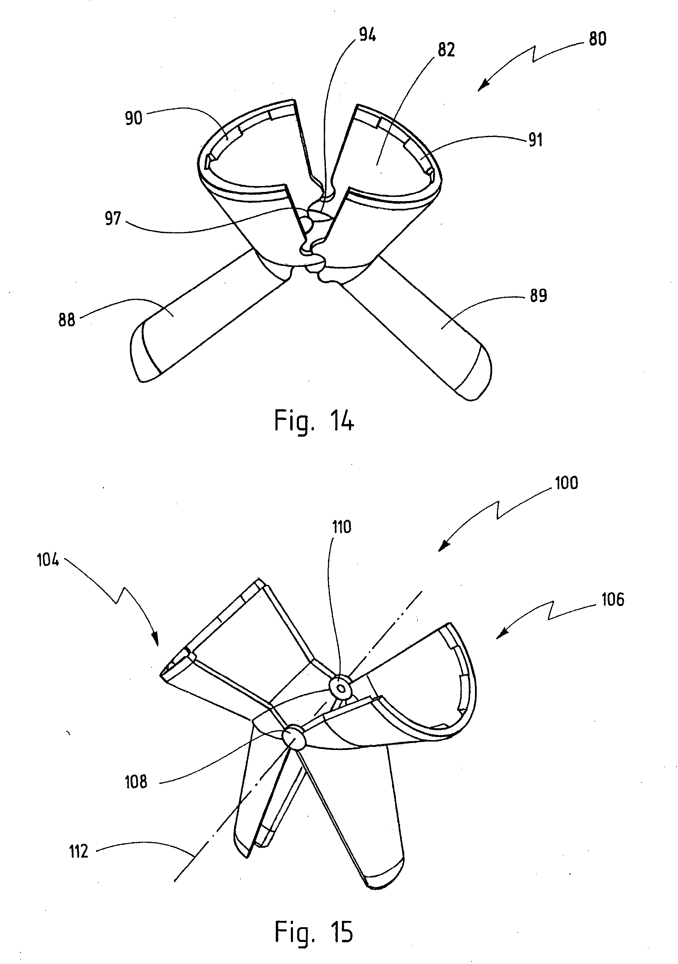

Bei dem in Fig. 15 dargestellten vierten Ausführungsbeispiel eines medizinischen Instrumentes ist dies in seiner Gesamtheit mit der Bezugsziffer 100 bezeichnet.

Das medizinische Instrument 100 ist aus zwei Teilkörpern 104 und 106 zusammengesetzt, die über feste Drehgelenke 108 und 110 miteinander verbunden sind. Somit existiert eine definierte Schwenkachse 112, um die die beiden Teilkörper 104 und 106 bei den zuvor beschriebenen Verschwenkbewegungen zwischen der ersten und zweiten Position hin und her verschwenkbar sind. Ansonsten sind die beiden Teilkörper 104 und 106 prinzipiell wie die zuvor beschriebenen Körper ausgebildet.

Die festen Drehgelenke 108 und 110 können so ausgebildet sein, dass sie dennoch leicht lösbar sind, um die beiden dann voneinander gelösten Teilkörper 104 bzw. 106 besser reinigen und sterilisieren zu können.

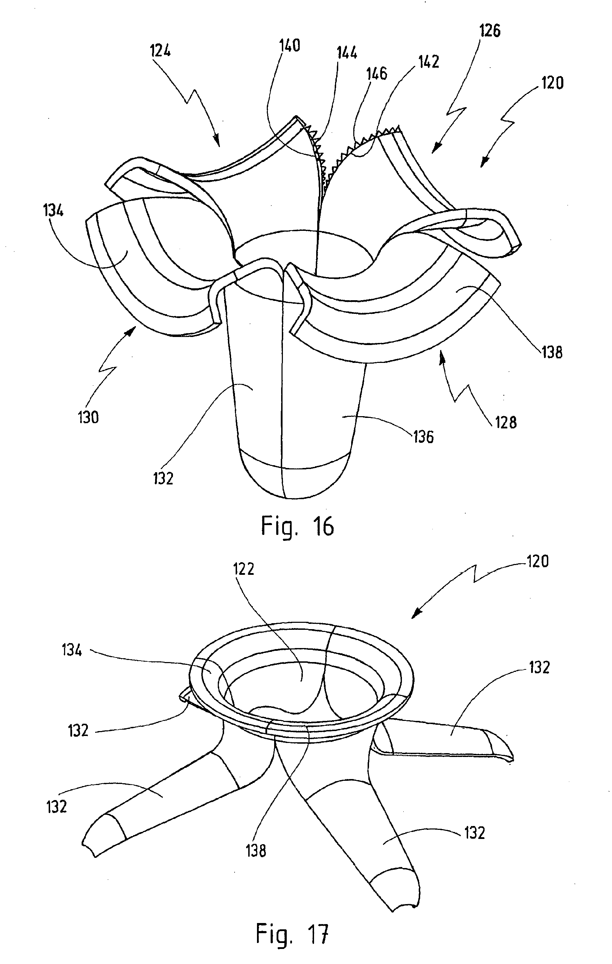

Bei dem in Fig. 16 und 17 dargestellten weiteren fünften Ausführungsbeispiel eines erfindungsgemäßen medizinischen Instrumentes ist dieses in seiner Gesamtheit mit der Bezugsziffer 120 bezeichnet.

Auch das medizinische Instrument 120 ist als ein Hohlkörper 122 ausgebaut, der hier aus vier Teilkörpern 124, 126, 128 und 130 zusammengesetzt ist.

Jeder einzelne dieser Teilkörper weist dann wieder einen entsprechenden distalen Teilkörperabschnitt 132 und einen davon abgewinkelten proximalen Teilkörperabschnitt 134 auf, wobei dies hier der Einfachheit halber nur bei dem Teilkörper 130 bezeichnet ist. Im Prinzip ist auch dieses medizinische Instrument 120 wieder gleich aufgebaut, es sind eben nur vier Teilkörper vorhanden, die in der ersten Position, wie sie in Fig. 16 dargestellt ist, so zusammengelegt werden können, dass ein distaler Hohlkörper 136 entsteht, der dann, wie zuvor mehrfach beschrieben, über eine Inzision in einen Körper einschiebbar ist.

Auch hier liegen die vier distalen Teilkörperabschnitte jeweils über ihre beiden Kantenflächen an entsprechenden benachbarten Teilkörperabschnitten an.

Beim Übergang von der in Fig. 16 dargestellten ersten Position in die in Fig. 17 dargestellte zweite Position, wird wieder die zuvor beschriebene Verschwenkbewegung durchgeführt, so dass dann der in Fig. 17 resultierende Körper entsteht, der einen proximalen Hohlkörper 138 aufweist, von dem die vier gespreizten distalen Teilkörperabschnitte 132 abstehen.

Wie aus Fig. 16 zu erkennen, sind an den Kantenflächen, hier an den Kantenflächen 140, 142 von zwei anliegenden Teilkörperabschnitten 124 und 126 dargestellt, entsprechend komplementäre schräge Verzahnungen 144 und 146 vorhanden, die die Abrollbewegung der Teilkörper bei dem Verschwenken aus der in Fig. 16 zu der in Fig. 17 dargestellten Position führen. Auch hier kann dann wieder in der Fig. 17 dargestellten Position eine entsprechende Kappe aufgesetzt werden.

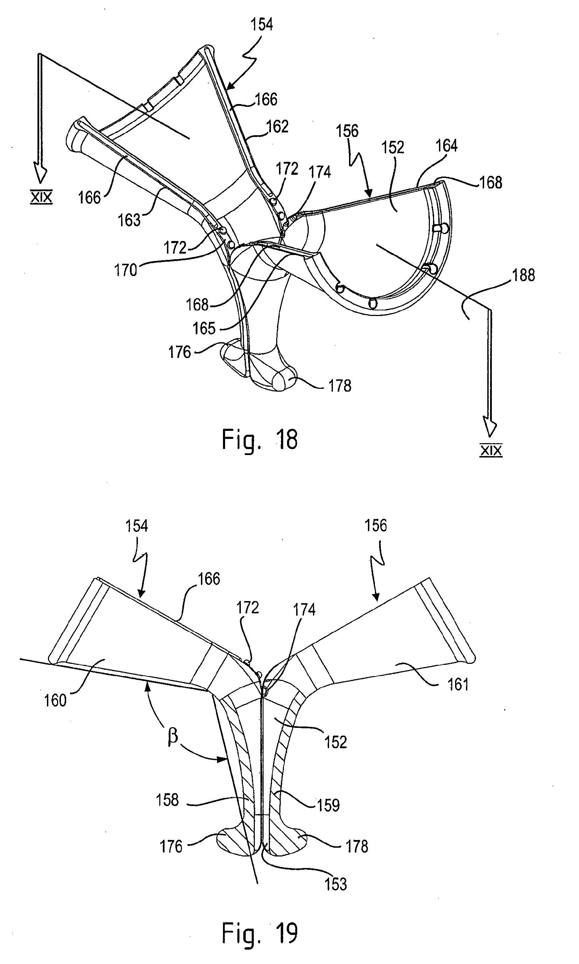

In den Fig. 18 bis 23 ist ein sechstes Ausführungsbeispiel eines medizinischen Instrumentes dargestellt, das in seiner Gesamtheit mit der Bezugsziffer 150 bezeichnet ist.

Aus den Fig. 18 bis 20 ist zu erkennen, dass auch das medizinische Instrument 150 als Hohlkörper 152 ausgebildet ist.

Der Hohlkörper 152 ist wieder aus zwei gegenüberliegenden Teilkörpem 154 und 156 zusammengesetzt.

Jeder der beiden Teilkörper 154 und 156 weist wieder einen distalen Teilkörperabschnitt 158 bzw. 159 und ein gegenüber diesem nach außen abgewinkelten proximalen Teilkörperabschnitt 160 und 161 auf, wie das zuvor beschrieben wurde.

Bei diesem Ausführungsbeispiel weisen die Kantenflächen 162 und 163 des Teilkörpers 154 jeweils eine Feder 166 auf. Die Kantenflächen 164, 165 des Teilkörpers 156 weisen jeweils Nuten 168 auf.

Das heißt, die beiden Teilkörper 154, 156 sind unterschiedlich ausgestaltet. Wenn diese beiden Teilkörper 154 und 156 längs deren Kantenflächen aneinandergefügt werden, stehen die Federn 166 des Teilkörpers 154 den Nuten 168 des Teilkörpers 156 gegenüber.

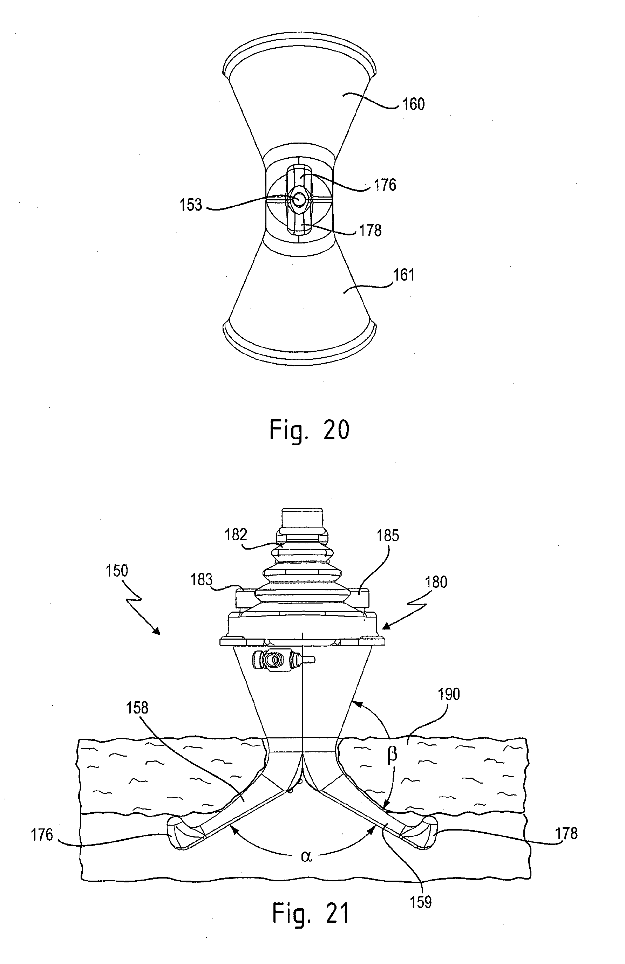

Dadurch wird ein seitliches Abgleiten verhindert. Außerdem sind die Materialwahl und die Gestaltung von Nut und Feder so ausgewählt, dass dadurch auch ein gasdichter Abschluss zwischen den proximalen Teilkörpern 160 und 161 besteht, wenn diese aneinandergefügt sind, wie das in Fig. 21 bis 23 ersichtlich ist.

Im Übergangsbereich 170 vom distalen zum proximalen Teilkörperabschnitt sind am Teilkörper 154 Vorsprünge 172 vorgesehen, die in gegenüberliegende Vertiefungen 174 am anderen Teilkörper 156 eingreifen.

Diese Vorsprünge 172 und Vertiefungen 174 führen beim Verschwenken der beiden Teilkörperabschnitte 154 und 156 zu einer exakten Führung, sie verhindern zusätzlich ein seitliches Abgleiten und stellen zusätzliche Dichtungsmaßnahmen in diesem Bereich dar.

Vom distalen Ende jedes distalen Teilkörperabschnittes 158 bzw. 159 steht nach außen gerichtet jeweils eine Nase 176 bzw. 178 vor.

Die Nasen 176, 178 stehen etwa rechtwinklig von der Mittellängsachse des distalen Hohlkörpers 152 vor und erstrecken sich in Richtung der proximalen Teilkörperabschnitte 160, 161, wie das insbesondere aus der Schnittdarstellung von Fig. 19 ersichtlich ist.

Aus dieser Schnittdarstellung ist auch ersichtlich dass bei aneinandergefügten distalen Teilkörperabschnitten 158, 159 eine Durchgangsöffnung 153 existiert, so dass bspw. schon in diesem Zustand ein Beobachtungsinstrument, bspw. ein Endoskop, hindurchgeführt werden könnte.

Wie aus den Fig. 21 bis 23 zu entnehmen, ist auf den distalen Rand der zusammengefügten proximalen Teilkörperabschnitte 160 und 161 ein Deckel in Form einer Kappe 180 aufgesetzt. Die Kappe 180 als solche kann aus elastischem Material bestehen, so dass diese selbst abdichtend wirkt, sie kann, wenn sie aus steiferen Materialien hergestellt werden kann, eine zusätzliche Dichtung aufweisen.

Aus den Darstellungen von Fig. 21 bis 23 ist zu entnehmen, dass von der Kappe 180 zwei gegenüberliegende Faltenbalgdome 181 und 182 hochstehen, zwischen denen noch drei zusätzliche Dichtungsöffnungen 183, 184 und 185 vorhanden sind.

Die äußeren Enden dieser Dichtungen sind als selbstschließende Schlitzdichtungen ausgeführt, so dass, falls durch diese keine schaftartigen Instrumente hindurchgeschoben sind, diese nach außen abdichten.

Ein seitlich angebrachter LUER-Anschluss 186 ermöglicht es, durch das medizinische Instrument 150 ein Gas in den Körper einzuführen, bspw. um diesen aufzublähen, so dass eine Inspektion des Innenraumes erleichtert wird. Genau dann ist es sehr wichtig, dass die Kappe 180 und die an dieser vorhandenen Dichtungsöffnungen auch bei eingeschobenen Instrumenten zur Außenseite hin abdichten. Eine Abdichtung wird auch, wie zuvor erwähnt, längs der Fügungskanten der proximalen Teilkörperabschnitte 160, 161 durch die zuvor erwähnte Nut- und Federkonstruktion sichergestellt.

Der Spreizwinkel α der gespreizten distalen Teilkörperabschnitte 158, 159 beträgt etwa 125°, die vorspringenden Nasen 176, 178 stützen sich dabei an der Unterseite der Bauchdecke 190 ab und fungieren hier als zusätzliche Kippsicherung.

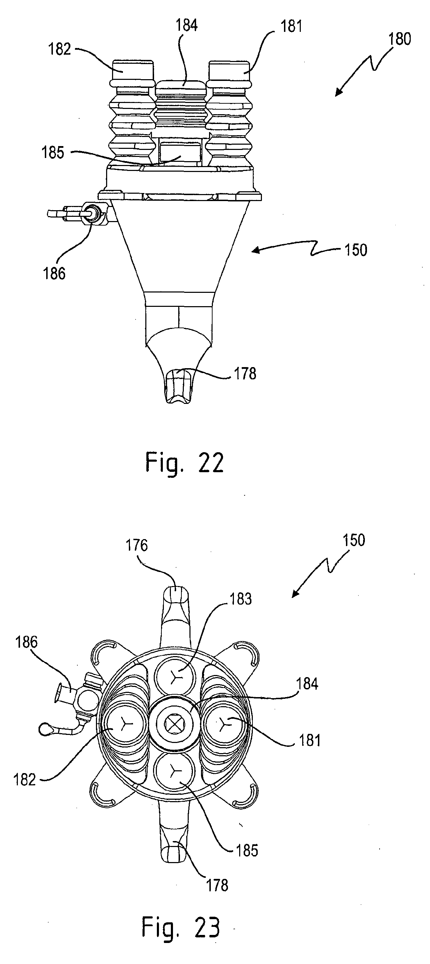

Wie aus der Draufsicht von Fig. 23 ersichtlich, können nun bis zu fünf schaftartige Instrumente durch die verschiedenen Dichtungsöffnungen 181-185 hindurchgeschoben werden. Aufgrund der domartigen Ausbildung der Faltenbalgdome 181 und 182 können diese Instrumente extrem gekippt werden, da die Domstruktur diesen Kippbewegungen folgen kann.

Somit können durch ein Instrument 150, das durch eine einzige Inzision in einen Köper eingeführt ist, zahlreiche schaftartige Instrumente eingeschoben werden. Diese können zusätzlich extrem gekippt werden, so dass in einem weiten Handhabungsbereich im Innern des Körpers bei nur einer Eingangsöffnung Manipulationen durchgeführt werden können.

Dies ist als ein besonderer Vorteil dieser Konstruktion als "Single Port Access" zu sehen.

Aus Fig. 19 und 21 ist zu ersehen, dass ein Winkel β, der sich zwischen einer äußeren Längskante eines proximalen Teilkörperabschnittes 160 und dem diesem zugehörigen distalen Teilkörperabschnitt 158 erstreckt, ebenfalls mehr als 90° beträgt. Dieser Winkel liegt in der Verschwenkebene 188, die in etwa der Schnittebene längs der Linien XIX-XIX in Fig. 18 entspricht.

Bei solchen Winkeln β > 90° kann die Handhabungsperson einen proximalen Teilkörperabschnitt eines Teilkörpers 154 oder 156 von Hand ergreifen und dessen Nase an der Inzision ansetzen, wobei diese Handhabung nicht durch die Oberseite der Bauchdecke 190 gestört oder gehindert wird. Dadurch ist ein einzelner Teilkörper 154 bzw. 156 sehr rasch und einfach einzubringen. Diese können dann im Körper unter Zuhilfenahme der Nut- und Federstruktur einfach aneinandergesetzt werden, und zwar zu einem Gebilde, wie es bspw. in Fig. 18 und 19 dargestellt ist. Zum Verschwenken können dann ebenfalls die vom Körper abstehenden proximalen Teilkörperabschnitte 160, 161 bspw. von Hand ergriffen und durch die Nut- und Federstruktur in Zusammenhang mit den Erhebungen und Vertiefungen zielgerecht in die in Fig. 21 bis 23 dargestellte Position verschwenkt werden. Die Vorsprünge 172 und die Vertiefungen 174 unterstützen diese zielgerichtete Abrollbewegung im Übergangsbereich 170.

Nach Aufstülpen der Kappe 180 ist das medizinische Instrument 150 einsatzbereit. Steht kein ausreichender Sichtraum zur Verfügung, kann zunächst über den LUER-Anschluss 186 ein Gas zugeführt werden, um den inneren Hohlraum aufzublähen. Anschließend können dann bis zu fünf schaftartige Instrumente durch die verschiedenen Dichtungsöffnungen der Kappe 180 durchgeführt werden, um einen minimalinvasiven Eingriff durchzuführen. Wird ein schaftartiges Instrument abgezogen, schließt die Dichtungsöffnung wieder gasdicht ab. Nach dem Einsatz wird die Kappe 180 abgenommen und die beiden Teilkörper 154, 156 können zusammen oder einzeln vom Körper wieder abgezogen werden.

## Patentansprüche

1. Medizinisches Instrument zum Schaffen eines Zuganges für einen minimalinvasiven Eingriff, mit einem Körper (12, 52, 82, 102, 122), der über eine Mittellängsachse (22, 62, 99) gesehen, aus zumindest zwei Teilkörpern (14, 16; 54, 56; 84, 86; 104, 106; 124, 126, 128, 130; 154, 156) zusammengesetzt ist, wobei jeder Teilkörper (14, 16; 54, 56; 84, 86; 104, 106; 124, 126, 128, 130; 154, 156) einen distalen Teilkörperabschnitt (18, 19; 58, 59; 88, 89; 132; 158, 159) aufweist, der in einen von der Mittellängsachse (22, 62, 99) nach außen abstehenden proximalen Teilkörperabschnitt (20, 21; 60, 61; 90, 91; 134; 160, 161) übergeht, wobei die distalen Teilkörperabschnitte (18, 19; 58, 59; 88, 89; 132; 158, 159) in einer ersten Position zu einem distalen Körper (32) mit seitlich abstehenden proximalen Teilkörperabschnitten (20, 21; 60, 61; 90, 91; 134; 160, 161) zusammenfügbar sind, und die proximalen Teilkörperabschnitte (20, 21; 60, 61; 90, 91; 134; 160, 161) in einer zweiten Position zu einem proximalen Hohlkörper (34) mit seitlich abstehenden distalen Teilkörperabschnitten (18, 19; 58, 59; 88, 89; 132; 158, 159) zusammenfügbar sind, wobei ein Übergang von der ersten Position zur zweiten Position und umgekehrt durch Verschwenken der Teilkörper bewerkstelligbar ist, und dass die Teilkörperabschnitte, in Richtung der Mittellängsachse (22, 62, 99) gesehen, Kantenflächen (24, 25; 64, 65; 66, 67; 140; 162-165) aufweisen, längs derer die Teilkörper zusammenfügbar sind, **dadurch gekennzeichnet, dass** die Kantenflächen Verzahnungsmerkmale aufweisen, die ein seitliches Abgleiten der Teilkörper quer zu den Kantenflächen sperren.

2. Medizinisches Instrument nach Anspruch 1, **dadurch gekennzeichnet, dass** die Kantenflächen (64, 65; 66, 67; 162-165) anliegender Teilkörper eine Nut- und Feder-Struktur (68; 70; 166, 168) aufweisen.

3. Medizinisches Instrument nach Anspruch 1 oder 2, **dadurch gekennzeichnet, dass** die Kantenflächen (24, 25) eines Teilkörpers Vorsprünge (28; 172) und Vertiefungen (30; 174) aufweisen, die mit entsprechenden Vertiefungen und Vorsprüngen an den Kanten des anderen anliegenden Teilkörpers in Eingriff treten.

4. Medizinisches Instrument nach einem der Ansprüche 1 bis 3, **dadurch gekennzeichnet, dass** die zusammengefügten distalen Teilkörperabschnitte (18, 19; 58, 59; 88, 89; 132; 158, 159) einen stabartigen distalen Körper (32) ergeben, dessen Durchmesser geringer ist als der aus den zusammengesetzten proximalen Teilkörperabschnitten (20, 21; 60, 61; 90, 91; 134; 160, 161) resultierende proximale Hohlkörper (34).

5. Medizinisches Instrument nach Anspruch 4, **dadurch gekennzeichnet, dass** der stabartige distale Körper (32) sich von distal nach proximal aufweitet.

6. Medizinisches Instrument nach einem der Ansprüche 1 bis 5, **dadurch gekennzeichnet, dass** die zusammengefügten proximalen Teilkörperabschnitte mit einem nach proximal abschließenden Deckel versehen sind.

7. Medizinisches Instrument nach Anspruch 6, **dadurch gekennzeichnet, dass** der Deckel als auf einen proximalen Rand des proximalen Hohlkörpers (34) aufgesetzte Kappe (40; 180) ausgebildet ist.

8. Medizinisches Instrument nach Anspruch 7, **dadurch gekennzeichnet, dass** die Kappe (40; 180) eine Dichtung (42) aufweist, die den proximalen Hohlkörper (34) nach proximal abdichtet.

9. Medizinisches Instrument nach Anspruch 7 oder 8, **dadurch gekennzeichnet, dass** die Kappe (180) mehrere Dichtungsöffnungen (181-185) aufweist, durch die schaftartige Instrumente dichtend hindurchführbar sind.

10. Medizinisches Instrument nach einem der Ansprüche 1 bis 9, **dadurch gekennzeichnet, dass** an der Außenseite (72) der distalen Teilkörperabschnitte (58, 59) Außengewindeabschnitte (74) angeformt sind.

11. Medizinisches Instrument nach einem der Ansprüche 1 bis 10, **dadurch gekennzeichnet, dass** der Übergang von der ersten Position in die zweite Position und umgekehrt durch ein Verschwenken der Teilkörper (14, 16; 54, 56; 84, 86; 104, 106; 124, 126, 128, 130; 154, 156) erfolgt, wobei sich die zu einem Körper zusammengefügten Teilkörperabschnitte voneinander wegbewegen und die vormals nach außen abstehenden Teilkörperabschnitte zu einem Körper zusammengefügt werden.

12. Medizinisches Instrument nach Anspruch 11, **dadurch gekennzeichnet, dass** die Verschwenkbewegung um eine Schwenkachse (112) im Übergangsbereich vom distalen zum seitlich abstehenden proximalen Teilkörperabschnitt (20, 21; 60, 61; 90, 91; 134) verläuft.

13. Medizinisches Instrument nach Anspruch 11 oder 12, **dadurch gekennzeichnet, dass** die Teilkörper (104, 106) über Drehgelenke (108, 110) aneinander gehalten sind, um deren Schwenkachse (112) die Schwenkbewegung erfolgt.

14. Medizinisches Instrument nach Anspruch 11 oder 12, **dadurch gekennzeichnet, dass** die Teilkörper (84, 86) im Bereich der Schwenkachse komplementäre Scharniergelenkmerkmale (92) aufweisen, die die Schwenkbewegung führen, ohne dass die Teilkörper (84, 86) fest miteinander verbunden sind.

15. Medizinisches Instrument nach einem der Ansprüche 1 bis 14, **dadurch gekennzeichnet, dass** vom distalen Ende eines distalen Teilkörperabschnittes (158,159) eine nach außen gerichtete Nase (176, 178) vorspringt.

16. Medizinisches Instrument nach einem der Ansprüche 1 bis 15, **dadurch gekennzeichnet, dass** ein Spreizwinkel α zwischen gespreizten distalen Teilkörperabschnitten mehr als 90° beträgt.

17. Medizinisches Instrument nach einem der Ansprüche 1 bis 16, **dadurch gekennzeichnet, dass** ein Winkel β zwischen einer äußeren Längskante eines proximalen Teilkörperabschnittes und des entsprechenden distalen Teilkörperabschnittes in einer Verschwenkebene (188) gesehen mehr als 90° beträgt.

## Claims

1. Medical instrument for creating an access for a minimally invasive intervention, with a body (12, 52, 82, 102, 122) which, seen along a central longitudinal axis (22, 62, 92) is assembled from at least two parts (14, 16; 54, 56; 84, 86; 104, 106; 124, 126, 128, 130; 154, 156) wherein each part (14, 16; 15, 56; 84, 86; 104, 106; 124, 126, 128, 130; 154, 156) has a distal section (18, 19; 58, 59; 88, 89; 132; 158, 159) which merges into a proximal section (20, 21; 60, 61; 90, 91; 134; 160, 161) standing outwardly from the central longitudinal axis (22, 62, 99) wherein the distal sections (18, 19; 58, 59; 88, 89; 132; 158, 159), in a first position can be joined to form a distal body (32) with the proximal sections (20, 21; 60, 61; 90, 91; 134; 160, 161) standing outwardly, and wherein the proximal sections (20, 21; 60, 61; 90, 91; 134; 160, 161), in a second position, can be joined to form a proximal hollow body (34) with the distal sections (18, 19; 58, 59; 88, 89; 132; 158, 159) standing outwardly, wherein a change-over from said first position to said second position, and vice versa, can be brought by pivoting said parts, and wherein the sections, viewing along the central longitudinal axis (22, 62, 29) are provided with edges (24, 25; 64, 65; 66, 67; 140; 162-165) along which the parts can be joined, **characterized in that** the edges are provided with interacting features which block a lateral sliding of said parts transversely to said edges.

2. Medical instrument of claim 1, **characterized in that** the edges (64, 65; 66, 67; 162-165) of joined parts have a grooves-and-tongues-structure (68; 70; 166, 168).

3. Medical instrument of claims 1 or 2, **characterized in that** the edges (24, 25) of a part is provided with protrusions (28; 172) and depressions (30; 174) which can engage with corresponding depressions and projections at the edges of the other joined part.

4. Medical instrument of any one of claims 1 through 3, **characterized in that** the joined sections (18, 19; 58, 59; 88, 89; 132; 158, 159) result in a rod-shaped distal body (32), the diameter of which is smaller than the proximal hollow body (34) resulting from the joined proximal sections (20, 21; 60, 61; 90, 91; 134; 160, 161).

5. Medical instrument of claim 4, **characterized in that** the rod-shaped distal body (32) widens from distal to proximal.

6. Medical instrument of any one of claims 1 through 5, **characterized in that** the joined proximal section are provided with a cover closing against a proximal side.

7. Medical instrument of claim 6, wherein said cover is designed as a cap (40; 180) fitted to a proximal rim of the proximal hollow body (34).

8. Medical instrument of claim 7, **characterized in that** the cap (40; 180) has a seal (42), sealing off the proximal hollow body (34) to the proximal side.

9. Medical instrument of claims 7 or 8, **characterized in that** the cap (180) is provided with several sealed openings (181-185), through which shaft-like instruments can be passed in a sealed manner.

10. Medical instrument of any one of claims 1 through 9, **characterized in that** an outer face (72) of the distal sections (58, 59) is provided with external thread sections (74).

11. Medical instrument of any one of claims 1 through 10, **characterized in that** the change-over from said first position into said second positions and vice versa is effected by pivoting said parts (14, 16; 54, 56; 84, 86; 104, 108; 124, 126, 128, 130; 154, 156) wherein the sections joined to a body move away from each other and the sections previously standing outwardly are joined to form a body.

12. Medical instrument of claim 1, **characterized in that** the pivoting takes place about a pivot axis (112) in the transition area from the distal to the standing outwardly proximally section (20, 21; 60, 61; 90, 91; 134).

13. Medical instrument of claims 11 or 12, **characterized in that** the parts (104, 106) are held on each other via swivel joints (108, 110) about the pivot axis (112) of which said pivoting takes place.

14. Medical instrument of claim 11 or 12, **characterized in that** the parts (84, 86) have, in an area of said pivot axis, complementary hinge-joint features (92), which guide said pivoting, without having the parts (84, 86) connected fixedly to each other.

15. Medical instrument of any one of claims 1 through 14, **characterized in that** a nose (176, 178) projects from a distal end of a distal section (158, 159) directed out-wardly.

16. Medical instrument of any one of claims 1 through 15, **characterized in that** a spreading angle α between the spreaded distal sections is more than 90°.

17. Medical instrument of any one of claims 1 through 16, **characterized in that** an angle β between an outer longitudinal edge of a proximal section and the respective distal section amounts more than 90° viewed in a pivoting plane (188).

## Revendications

1. Instrmnent médical destiné à créer un accès pour une intervention mini-invasive, comportant un corps (12, 52, 82, 102, 122) qui, vu selon un axe longitudinal médian (22, 62, 99), est composé d'au moins deux corps partiels (14, 16 ; 54, 56 ; 84, 86 ; 104, 106 ; 124, 126, 128, 130 ; 154, 156), où chaque corps partiel (14, 16 ; 54, 56 ; 84, 86 ; 104, 106 ; 124, 126, 128, 130 ; 154, 156) présente un segment de corps partiel distal (18, 19 ; 58, 59 ; 88, 89 ; 132 ; 158, 159) qui se convertit en un segment de corps partiel proximal (20, 21 ; 60, 61 ; 90, 91 ; 134 ; 160, 161) faisant saillie vers l'extérieur depuis l'axe longitudinal médian (22, 62, 99), où les segments de corps partiels distaux (18, 19 ; 58, 59 ; 88, 89 ; 132 ; 158, 159) peuvent être assemblés dans une première position en un corps distal (32) présentant des segments de corps partiels proximaux (20, 21 ; 60, 61 ; 90, 91 ; 134 ; 160, 161) en saillie latéralement, et où les segments de corps partiels proximaux (20, 21 ; 60, 61 ; 90, 91 ; 134 ; 160, 161) peuvent être assemblés dans une seconde position en un corps creux proximal (34) présentant des segments de corps partiels distaux (18, 19; 58, 59; 88, 89; 132 ; 158, 159) en saillie latéralement, où une transition de la première position vers la seconde position et inversement peut être réalisée par pivotement du corps partiel, et en ce que les segments de corps partiels, vus dans la direction de l'axe longitudinal médian (22, 62, 99), présentent des surfaces d'arête (24, 25 ; 64, 65 ; 66, 67 ; 140 ; 162, 165) le long desquelles les corps partiels peuvent être assemblés, **caractérisé en ce que** les surfaces d'arête présentent des caractéristiques de denture qui bloquent un glissement latéral des corps partiels transversalement aux surfaces d'arête.

2. Instrument médical selon la revendication 1, **caractérisé en ce que** les surfaces d'arête (64, 65 ; 66, 67 ; 162, 165) de corps partiels contigus présentent une structure de type rainure et languette (68 ; 70 ; 166, 168).

3. Instrument médical selon la revendication 1 ou 2, **caractérisé en ce que** les surfaces d'arête (24, 25) d'un corps partiel présentent des saillies (28 ; 172) et des creux (30 ; 174) qui viennent en prise avec les creux et saillies correspondants au niveau des arêtes de l'autre corps partiel contigu.

4. Instrument médical selon l'une des revendications 1 à 3, **caractérisé en ce que** l'assemblage des segments de corps partiels distaux (18, 19 ; 58, 59 ; 88, 89 ; 732 ; 158, 159) donne un corps distal en forme de baguette (32) dont le diamètre est plus petit que celui du corps creux proximal (34) découlant des segments de corps partiels proximaux (20, 21 ; 60, 61 ; 90, 91 ; 134 ; 160, 161) assemblés.

5. Instrument médical selon la revendication 4, **caractérisé en ce que** le corps distal en forme de baguette (32) s'élargit depuis la partie distale vers la partie proximale.

6. Instrument médical selon l'une des revendications 1 à 5, **caractérisé en ce que** les segments de corps partiels proximaux assemblés sont munis d'un couvercle qui se ferme vers la partie proximale.

7. Instrument médical selon la revendication 6, **caractérisé en ce que** le couvercle est exécuté en tant que chapeau (40 ; 180) placé sur un bord proximal du corps creux proximal (34).

8. Instrument médical selon la revendication 7, **caractérisé en ce que** le chapeau (40 ; 180) présente un joint (42) qui ferme de façon étanche le corps creux proximal (34) vers l'extrémité proximale.

9. Instrument médical selon la revendication 7 ou 8, **caractérisé en ce que** le chapeau (180) présente plusieurs ouvertures de joint (181-185) par lesquelles des instruments en forme de tige peuvent être introduits de façon étanche.

10. Instrument médical selon l'une des revendications 1 à 9, **caractérisé en ce qu'**au niveau du côté extérieur (72) des segments de corps partiels distaux (58, 59) sont formés des segments à filetage externe (74).

11. Instrument médical selon l'une des revendications 1 à 10, **caractérisé en ce que** la transition de la première position vers la seconde position et inversement se fait par un basculement des corps partiels (14, 16 ; 54, 56 ; 84, 86 ; 104, 106; 124, 126, 128, 130 ; 154, 156), les segments de corps partiels assemblés en un corps s'écartant les uns des autres et les segments de corps partiels précédemment en saillie vers l'extérieur s'assemblant en un corps.

12. Instrument médical selon la revendication 11, **caractérisé en ce que** le mouvement de basculement se fait autour d'un axe de pivotement (112) dans la zone de transition, de distal vers proximal en saillie, du segment de corps partiels (20, 21 ; 60, 61 90, 91 ; 134).

13. Instrument médical selon la revendication 11 ou 12, **caractérisé en ce que** les corps partiels (104, 106) sont retenus l'un à l'autre par des articulations tournantes (108, 110), le mouvement de basculement se faisant autour de l'axe de pivotement (112) de celles-ci.

14. Instrument médical selon la revendication 11 ou 12, **caractérisé en ce que** les corps partiels (84, 86) présentent dans la zone de l'axe de pivotement des caractéristiques d'articulation par charnière (92) complémentaires qui conduisent le mouvement de pivotement sans que les corps partiels (84, 86) soient reliés fermement ensemble.

15. Instrument médical selon l'une des revendications 1 à 14, **caractérisé en ce qu'**un taquet (176, 178) orienté vers l'extérieur fait saillie depuis l'extrémité distale d'un segment de corps partiel distal (158, 159).

16. Instrument médical selon l'une des revendications 1 à 15, **caractérisé en ce qu'**un angle d'écartement α entre les segments de corps partiels distaux écartés est supérieur à 90°.

17. Instrument médical selon l'une des revendications 1 à 16, **caractérisé en ce qu'**un angle β entre une arête longitudinale extérieure d'un segment de corps partiel proximal et le segment de corps partiel distal correspondant est supérieur à 90°, tel que vu dans un plan de pivotement (188).
